# EUROPEAN PATENT APPLICATION

(11) **EP 2 179 738 A2**
(43) Date of publication of application: **28.04.2010**
(21) Application number: 10000798.8
(22) Date of filing: 22.01.1998
(51) Int. Cl.: A61K 35/28, C12N 5/00

(54) **Use of mesenchymal stem cells for treating osteoporosis**

(30) Priority: 24.01.1997 US 36388 P
(62) Divisional of application: 98903613.2
(71) Applicant: Osiris Therapeutics, Inc., Baltimore, MD 21231-2001 (US); Case Western Reserve University, Cleveland, OH 44106 (US)
(72) Inventor: Bruder, Scott, P., Owings Mills, MD 21117 (US); Caplan, Arnold, I., Cleveland Heights, OH 44121 (US); Burns, James, S., Annapolis, MD 21403 (US); Marshak, Daniel, Lutherville, MD 21093 (US)
(74) Representative: Vossius, Volker

(57) **Abstract**

The osteoporosis treatment and infusion compositions of the invention increase the number of the patient's own progenitor cells (hMSCs) and the reservoir of available bone-forming osteoblasts. They can be used to generate cortical and cancellous bone which has been lost as a result of post-menopausal osteoporosis, male idiopathic osteoporosis, congenital osteoporosis and other bone resorption diseases. Most cases of osteoporosis can be treated with a metered intravenous infusion of the composition of the invention over the course of 15-30 minutes, using customary cell reinfusion therapy techniques.

## Description

### Background of the Invention

Osteoporosis is a progressive disease characterized by loss of bone mass and subsequent risk of battle bone fractures, resulting characteristically from the decrease in naturally occurring estrogen levels in post menopausal women. According to the National Osteoporosis Foundation, osteoporosis affects as many as 24 million post-menopausal women in the U.S. and an even higher number throughout Europe and Japan. Osteoporosis accounts for as many as 1.3-1.4 million bone fractures per year in the U.S., with as many as 4 million throughout the developed countries. inventors estimates that 75-80% of the over 300,000 elderly hip fractures which occur annually in the U.S. are associated with osteoporosis. These fractures are responsible for substantially greater morbidity and mortality than the fractures suffered by younger patients. As many as 25 to 30% of elderly hip fracture patients die within six months from complications related to their fractures. The direct and indirect cost of treating osteoporotic fractures is estimated to exceed U.S. $10-12 billion per year.

Over 40% of post-menopausal women between the ages of 45-70 show significant bone loss due to osteoporosis according to Biomedical Business International. And, by age 75, about 50% of both men and women are affected. The actual number of people affected will grow at a rate greater than simple population growth rates because the aging of the population is disproportionately increasing the older cohorts while the age for the onset of menopause has remained constant.

Hormone replacement therapy ("HRT") using estrogen and other products which stabilize the rate of bone loss, such as calcitonin and the bisphosphonates, are currently the most effective methods for preventing or treating osteoporosis in post-menopausal women. Approximately 11 million U.S. women over age 45 currently use hormone replacement therapy. The efficacy of HRT is not well measured for most treated women because bone density studies are not routinely performed. The methods of measurement include: duel energy X-ray absorbtiometry (DEXA) and computed tomography (CT) scans. In order to be useful in guiding therapy studies of the individual patient must be conducted over a period of time so that changes in bone density can be measured. A new urinalysis test for measurement of relative rate of bone loss has recently been approved for marketing. *Premarin* has dominated the HRT market with approximately 75% of the estrogen prescriptions.

Estrogen, calcitonins, bisphosphonates and other anti-resorption drugs are now the most prevalent methods of preventing or treating osteoporosis. These products act to inhibit the rate of bone resorption, thereby compensating for the reduced rate of bone regeneration after menopause.

However, despite the widespread availability of these drugs, it is believed that 10-15% or more of the osteoporotic patient population (in the U.S. approximately 2.4-3.6 million post-menopausal women) may either not be able to tolerate these medications or may be refractory to these therapeutic modalities.

The existing products are generally available and are easily utilized pharmaceutical formulations, they are not without problems. The recent publication in the *New England Journal of Medicine* of a large study that showed significantly higher incidence of breast cancer among women taking HRT and is, therefore, an important issue in the acceptability of this therapy.

In summary, there are currently no products that can effectively reverse bone loss resulting from osteoporosis.

### Summary of the Invention

Bone loss resulting from osteoporosis is believed to be a function of accelerated bone resorption due to reduced levels of circulating estrogens, while bone regeneration capacity has declined due to the normal effects of aging. The altered balance of resorption and osteogenesis results in significant bone loss. Current pharmacological approaches to redressing this imbalance rely on stimulating the declining pool of osteoblasts or inhibiting osteoclast-based bone resorption.

In accordance with an aspect of the present invention there is provided a process for treating osteoporosis by administering to a mammal in need thereof mesenchymal stem cells in an amount effective to treat osteoporosis.

The present invention is directed, *inter alia,* to the evolution of a regenerative tissue therapy using human mesenchymal stem cells (MSCs) for the treatment osteoporosis. This effort is aimed at developing a truly osteogenic therapy; that is, to create new mineralized bone tissue at critical sites rather than simply preventing the loss of existing bone. This approach to the problem is novel because it utilizes the replacement of the early progenitors of bone formation at the cellular level, rather than relying on endocrine manipulations or synthetic chemical supplements.

Systemic bone loss resulting from osteoporosis cannot generally be repaired via osteoconductive or osteoinductive treatments because patients, particularly post-menopausal women over the age of 50, are unable to recruit or generate sufficient bone-forming progenitor cells to heal the bone defect. By increasing the number of the patient's own mesenchymal progenitor cells (hMSCs) and subsequently increasing the reservoir of available bone-forming osteoblasts, bone mass can be restored and the risk of age-related fractures reduced, using the body's own natural repair mechanism.

The infusion preparations of the invention include a suspension of purified culture-expanded autologous bone marrow-derived human mesenchymal stem cells (hMSCs). These cells are the naturally occurring progenitors which give rise to multiple structural and connective tissues, including normal bone. The infusion preparations of the invention, unlike anti-resorption agents, hormone replacement therapy (HRT) and anti-estrogens, have the ability to regenerate new bone following post-menopausal bone loss due to the inclusion of purified autologous progenitor cells.

The method of treatment and infusion compositions of the invention address these issues directly by increasing the number of the patient's own progenitor cells (hMSCs) and increasing the reservoir of available bone-forming osteoblasts. They can be used, for example, in regenerating cortical and cancellous bone which has been lost as a result of post-menopausal osteoporosis, male idiopathic osteoporosis, congenital osteoporosis and other bone resorption diseases. Most cases of osteoporosis can be treated with a metered intravenous infusion of the composition of the invention over the course of 15-30 minutes, using customary cell reinfusion therapy techniques. This requires no special equipment or facilities and can be readily accomplished on an out-patient basis. Quarterly, semiannual or annual administration is usually required, depending on the severity of bone loss, pattern of bone restoration, and the maintenance of a proper osteoblast to osteoclast ratio.

The procedure starts with aspiration of approximately 10-20cc of bone marrow from the patient's medial posterior iliac crest using standard aseptic techniques. This typically yields 1,000-5,000 hMSCs. A bone marrow collection and transport kit as described herein provides all necessary materials to facilitate the harvest and assure that an appropriate and usable marrow aspirate is harvested.

A single harvest of bone marrow aspirate is culture expanded to generate 5-10 patient doses obtained from primary, first or second through fourth passage hMSCs. The patient's hMSCs are cryopreserved and can be maintained as viable cells for up to five years following harvest. Approximately 50-150 million culture-expanded autologous hMSCs are returned to the patient in the form of infusion preparations of the invention.

### Detailed Description of Preferred Embodiments

The term "treating" as used herein means reducing the severity of osteoporosis and/or delaying the onset of osteoporosis and/or reducing or slowing the progress thereof.

The mammal is preferably a human and the mesenchymal stem cells used for such treatment are human mesenchymal stem cells, and such human mesenchymal stem cells are preferably autologous cells.

The human mesenchymal stem cells may be used alone or in admixture with other cells; e.g., a preparation enriched for human mesenchymal stem cells.

In a preferred embodiment, the human mesenchymal stem cells are administered intravenously in a pharmaceutically acceptable carrier.

Human mesenchymal stem cells may also be administered at a site at which osteoporosis is occurring; e.g., injection at such site.

The infusion preparations of the invention are indicated, for example, for use in regenerating cortical and cancellous bone which has been lost as a result of post-menopausal osteoporosis, steroid-induced osteoporosis, renal dialysis-related or male idiopathic osteoporosis. Most osteoporotic defects will be treated with a slow-drip (or programmable pump) intravenous infusion over the course of 15-30 minutes, using customary cell reinfusion therapy protocols.

Infusion preparations in accordance with the invention preferably containing autologous hMSCs become osteogenic in vivo and, as such, regenerate bone tissue directly following their homing to the bone marrow where they are able to replicate and subsequently differentiate into bone-forming osteoblasts. This process is referred to as "regenerative tissue therapy". The direct activity of these mesenchymal progenitor cells is superior to osteoinductive, HRT or anti-resorption agents, in part, because current pharmaceutical approaches to the treatment of osteoporosis depend on inhibiting osteoclast activity, or activating a declining reserve of osteoblasts in the marrow. These approaches are not able to replenish the pool of bone-forming osteoblasts which differentiate from hMSCs. By contrast, the infusion preparations of the invention directly replenish the progenitor cells which have diminished as a result of estrogen deficiency, steroid excess, or simply normal aging.

All infusion procedures should be performed under standard aseptic cell reinfusion conditions, following accepted guidelines for reinfusing whole blood, platelets and other blood components. The human mesenchymal stem cells are maintained in a liquid suspension inside a reinfusion bag at between 20°C and 80°C (36°F and 46°F) until several hours prior to reinfusion. At that time the infusion preparation is brought to room temperature for the stem cell reinfusion. All aspects of the reinfusion should be performed in accordance with accepted standards of intravenous infusion management.

Frequency of administration can be quarterly, semi-annually or annually, depending on the severity of bone loss, pattern of bone restoration, and maintenance of a proper post-infusion ob:oc ratio. Dose and frequency of administration should be adjusted to reflect the above considerations. In general, each marrow aspirate will generate sufficient hMSCs to prepare and store 5-10 patient doses, obtained from primary, 1st passage or 2nd through 4th passage hMSCs inventors will cryopreserve and maintain patients' hMSCs for up to five (5) years following receipt of the initial marrow aspirate, after which a fresh marrow aspirate will be required to continue a patient's course of therapy.

The infusion preparations of the invention are contraindicated (1) in sites with significant vascular impairment proximal to the infusion site, (2) in the presence of other nonosteoporotic systemic bone disorders, (3) in conjunction with high blood pressure or other cardiovascular conditions, (4) in the presence of moderate to severe respiratory conditions, (5) where the patient has a known allergy to one or more infusion components, (6) in the presence of other contraindicated drugs such as dexamethasone, anti-emetics, or (7) in patients known to have any blood dyscrasias.

The current clinical assessment of osteoporosis is based on Dex scans and Bone Mineral Density measurements. A diagnosis of osteoporosis is usually based on 2.5 standard deviations below average bone density. Bone Mineral Density, while a fairly accurate measurement of bone mass, does not reflect the patient's osteogenic capacity, nor does it reflect the concentration of osteoprogenitor cells required to restore the pre-menopausal balance between bone formation and bone resorption.

The invention further provides a new clinical osteogenic assay to augment the measurement of bone mineral density. This test is referred to as the "bone formation index", or the ob:oc ratio. This assay measures the relative concentration of osteoblasts (or osteoblast precursors) and osteoclasts, as determined by the measurement of surface antigens present on various bone forming and bone resorbing cells in the bone marrow. The measurement of mesenchymal stem cells, with or without the use of another cell or molecular marker, can be used for the measurement of bone density or osteogenic capability.

Mesenchymal stem cells regenerate new bone based on increasing the patient's osteoblastic activity to achieve a balance relative to the patient's osteoclastic activity, measured by the ratio of osteoblasts to osteoclasts, or ob:oc ratio, measured over time both before and subsequent to periodic hMSC therapy. Following each infusion administration, substantial new bone is formed in 6-10 weeks. By 12 to 16 weeks, additional bone is formed and a more normal pattern of bone formation and resorption (turnover) is underway. The regenerated bone is incorporated into the patient's host bone, while the addition of new mesenchymal progenitor cells (hMSCs) in the bone marrow support the normal remodeling process and ensures long-term structural integrity.

A low ob:oc ratio (in the range of 0.05-0.35) indicates a serious deficiency in osteogenic activity with a correspondingly high risk of long-bone, hip or pelvic fractures. A low ob:oc ratio may also be indicative of advanced osteoporosis, rapidly progressing disease, or patients who are refractory to current therapies such as estrogen replacement, calcitonin, or various anti-resorption agents. Moderate or high ob:oc ratios reflect earlier or more benign stages of the disease where osteoblast. Reserves have not been irreversibly depleted, and lower doses of osteoprogenitor cells or less frequent administration may return the patient to a more normal balance of bone formation and bone resorption.

The infusion treatment regimen and preparations of the invention represent a novel approach to the treatment of disease through a unified system of patient diagnosis, treatment and follow-through care. Starting from an initial autologous patient sample (10-20 ml marrow aspirate), the clinician can now diagnose the patient's disease status, determine dosing and frequency regimens, administer the infusions, and store further doses for subsequent reinfusions.

The infusions are prepared using the patient's own (autologous) cells which have been previously harvested. The cells are culture-expanded for approximately 3-6 weeks after harvest, until 1-2 days prior to the scheduled "regenerative tissue therapy" reinfusion.

### Mesenchymal Stem Cell Harvest and Culture-Expansion

Using the bone marrow collection and transport kit (described below), a bone marrow aspirate from the medial posterior iliac crest is obtained by standard aseptic techniques in an out-patient procedure. A minimum sample size of 10-20cc, which may vary depending on patient age, is required to assure an adequate concentration of hMSCs in the primary cultures. Since hMSCs decline with age, it is important to obtain the proper starting stem cell concentration.

Nucleated cells are harvested from the bone marrow and subsequently processed in individual batches under sterile culture conditions which promote selective attachment of the rare mesenchymal stem cells. Typically, only 100 to 500 hMSCs per 10-50 million nucleated marrow cells (or fewer in the case of osteoporotic patients) attach and grow in tissue culture. This translates to approximately 5,000 hMSCs per 10cc marrow aspirate. The remainder of the cell population contains various types of non-adherent hematopoietic and stromal cells which are removed early in the cell culturing procedure.

Adherent marrow-derived hMSCs have homogeneous morphology, almost all being fibroblastic, with rare, polygonal cells. The adherent cells are seen as small colonies of only a few cells on day 3; however, they replicate rapidly and form colonies of 50-200 cells within the first week of culture. By 10-14 days, the colonies of mesenchymal stem cells have expanded in size with each colony containing several hundred to several thousand cells.

To maintain mesenchymal stem cells in their undifferentiated state and to control their rate of replication, each primary hMSC culture is passaged into new culture vessels when the culture becomes about 80-90% confluent. Cells in newly passaged cultures attach to form a uniformly distributed layer of cells which are 25-35% confluent at the time they are passaged, Cells continue to divide and are again passaged when cell density reaches about 80-90% confluence, yielding an average of about 5 million cells per T-flask culture vessel.

The hMSC preparations are preferably culture-expanded in a chemically-defined medium which does not require the addition of fetal calf serum or other animal or human serum supplements. This medium is the subject of copending, commonly assigned U.S. patent application serial no. 08/464,599 entitled "Chemically Defined Medium for Human Mesenchymal Stem Cells, filed June 5, 1995.

Cells from each culture vessel can be replated many times without a loss in the osteogenic potential of the cells. Therefore, a single primary culture starting with 100 to 500 adherent human mesenchymal stem cells can be expanded to well over one billion (10⁹) cells. Typically, however, a small 10-20cc arrow aspirate provides 25 primary culture vessels of up to 5 million cells, and consequently, sufficient cells for most infusions can be obtained in fewer than 2-3 passages. Purification and culture-expansion of the patient's autologous mesenchymal stem cells can take from 3-8 weeks, depending on the number of primary hMSCs in the patient's marrow aspirate and the number of cells required for the infusion. As a result, the "regenerative tissue therapy" procedure should be scheduled with this timetable in mind.

Bone marrow collected with the bone marrow collection and transport kit described herein should be processed according to the protocol described herein.

### Bone Marrow Collection and Transport Kit

Prep Tray: povidone iodine swab sticks (1% available iodine) (3); paper towel; fenestrated drape; and hospital drape.

Procedure Tray: Jamshidi Bone Marrow Biopsy/Aspiration Needle, 4"; Illinois sternal/iliac aspiration needle, 15GA; bone marrow transport vessel, 20cc; 10cc syringe, Luer slip; 20cc syringe, Luer slip; 5cc syringe, with 20GA x 1 1/2" needle; 10cc syringe, Luer slip; 21 GA x 1 1/2" needle; 25GA x 5/8" needle; lidocaine hydrochloride USP, 1%, 5ml (2) ; heparin USP, (?)10,000 U/ml, 5ml; scalpel blade with handle; gauze sponges (5); elastic bandage; probe; and plastic bags, (2).

Stem Cell Transport Container: protective wrap for transport vessel; plastic bag for ice; and three (3) cold blocks.

### Infusion Kit

Infusion suspension of autologous hMSCs, premeasured in sterile i.v. bags (50 million, 100 million, 150 million, or custom reinfusions of 200+ million hMSCs) i.v. reinfusion adapter (for connecting autologous cell suspension to i.v. lines) . Infusion preparations should be stored at refrigerated conditions between 20°C and 80°C (36°F and 46°F).

The numerous aspects of osteoporosis therapy contemplated include those described in more detail below.

The present inventors and their colleagues have studied extensively the MSCs in aspirates from the iliac crest marrow of nearly 800 patients and healthy volunteers. Of these, a substantial portion were post-menopausal women including many over the age of 70. MSCs were obtained from all marrows sampled, indicating that these cells are present in the marrow of post-menopausal and elderly patients, but the number of MSC colony forming units declines with age. Complete clinical histories, including estrogen treatments and pregnancies were not recorded; however, the numbers of MSC colonies that formed in primary culture were recorded for most samples.

The present studies involve obtaining peripheral blood samples (50-200 ml), iliac crest bone marrow aspirates (10-20 ml), and iliac crest bone biopsies. The types of cells to be analyzed and cultured from these samples are: (1) marrow-derived MSCs; (2) bone-derived osteoblasts; (3) peripheral blood-derived pre-osteoblasts; (4) marrowderived osteoclasts; (5) peripheral blood-derived monocytes and myeloid progenitors. The studies examine cell number in each category according to several parameters. Normal, healthy male and female volunteers with no clinical diagnosis of osteoporosis (age 25-50) are sampled for crest marrow, iliac bone and peripheral blood to isolate and culture the cell populations described above.

Female patients are studied to determine the effects of estrogen depletion on cell numbers. Clinical histories of estrogen treatment, menarche, menopause, pregnancies, and live births are obtained to help sort the data. In many cases, for example, a percentage of the patients are taking estrogen replacement therapies, and some younger patients are taking or have a history of taking synthetic estrogens for birth control. Significant correlations on the effect of estrogen depletion on MSC number can be garnered from population sizes of several hundred to a few thousand, since the clinical histories will vary. First, statistically significant populations of premenopausal and post-menopausal women are compared within a relatively narrow age range in the fourth to fifth decade. Second, patients scheduled for ovariectomy are sampled before and one to three years after the procedure to determine the changes in cell number from the same individual. Third, patients with a family history of osteoporosis are sampled, and correlations drawn separately from other groups.

The isolation and culture expansion of hMSCs is described in U.S. Patent No. 5,486,359.

### Preclinical Rat Model of Osteoporosis

Rats were ovariectomized and maintained for 7 weeks to establish significant osteopenia. The ovariectomized rats were subsequently infused intravenously with approximately 10⁶ culture expanded MSCs, and animals were sacrificed 35 days later. The results are summarized in Table 2.

**TABLE 2**

| **Restoration of Bone Loss in Ovariectomized Rats is Achieved by Intravenous Infusion of Syngeneic Mesenchymal Stem Cells** | | | |
|---|---|---|---|
| **Bone Formation Parameter** | **Control Group** | **Restoration Group** | **P value** |
| Bone Mineral Content (mg) | 1.278±0.052 | 1.331±0.037 | <0.005 |
| Central femur | | | |
| Mineralized Bone area (cm²) | 0.610±0.015 | 0.621±0.009 | <0.02 |
| Distal femur | | | |
| Bone Mineral Density (mg/cm²) | 157.7±14.97 | 160.4±11.38 | >0.8 |
| Distal femur | 137.0±9.73 | 139.1±4.99 | >0.6 |
| Central femur | | | |
| Mineralizing Surfaces (%) | 14.02±8.14 | 21.17±8.44 | <0.04 |
| Double Label Only | 17.60±8.20 | 25.50±8.26 | <0.02 |
| Double + Half Single Label | | | |
| Bone Formation Rate (mm²/year) | 0.540±0.236 | 0.903±0.597 | <0.04 |

DEXA scans and quantitative histomorphometry of the femur indicate significant increases in diaphyseal and metaphyseal bone volume and mass in experimental groups receiving MSCs. Furthermore, bone density was equivalent to normal control rats indicating that the new bone formed was indeed similar to normal trabecular and cortical bone. No estrogen supplementation was performed on these animals. In addition, results from additional studies suggest that direct, diaphyseal injection of MSCs results in enhanced bone regeneration in OVX rats over intravenous administration of the cells.

### Literature Cited

1. Marie, P.J. Human osteoblastic cells: A potential tool to assess the etiology of pathologic bone formation., J Bone Miner. Res. 12:1847-1850, 1994.
2. Parfitt, A.M., Drezner, M.K., Glorieux, F.H., Kanis, J.A., Malluche, H., Meunier, P.J., Ott, S.M., Recker, R.R. Bone histomorphometry: Standardization of nomenclature, symbols and units. J Bone Miner. Res., 2:595-610, 1987.
3. Seeman, E., Tsalamandris, C., Bass, S., Pearce, G. Present and future of osteoporosis therapy. Bone, 17:23S-29S, 1995.
4. Marie, P.J., Sabbagb, A., de Vemejoul, M.-C., Lonui, A. Osteocalcin and deoxyribonucleic acid synthesis in vitro and histomorphometric indices of bone formation in postmenopausal osteoporosis., J Clin. EndocrinoL Metab., 69:272278,1989.
5. Marie, P.J. Human endosteal osteoblastic cells: relationship with bone formation. Calcif. Tissue Int. pp. S 13- S 16, 1995.
6. Haynesworth, S.E., Goshima, J., Goldberg, V.M., Caplan, A.I. Characterization of cells vath osteogenic potential from human marrow. Bone, 13, 81-88, 1992.
7. Bruder, S.P., Lawrene, E.G., Haynesworth, S.E. Identification and characterization of human osteogenic cell surface differentiation antigens. J Bone Miner. Res. 10:(Suppl 1) Abstract T249, S416, 1995.
8. Long, M.W. Williams, J.L. Mann, KG. Expression of human bone-related proteins in the hematopoietic microenvironment. J Clin. Invest. 86:1387-1395, 1990.
9. Purton, L.E., Torok-Storb, B. Normal human peripheral blood mononuclear cells mobilized with granulocyte colony-stimulating factor (G-CSF) contain osteoclast precursors. Blood (in press), 1996.
10. Haynesworth, S.E., Goldberg, V.M., Caplan, A.I. Diminution of the number of mesenchymal stem ceils as a cause for skeletal aging. In Buckwalter J.A., Goldberg, V.M. Woo, S.L.Y. (eds,): In Musculoskeletal Soft-Tissue Aging: Impact on Mobility. Rosemont: AAOS, Section 1, pp 80-86, 1994.
11. Caplan A.I. ne Mesengenic Process. Clin. Plast. Surg., 21:429-435, 1994
12. Stern, P.H., Cellular Physiology of Bone. In "Metabolic Bone and N4ineral Disorders" (S.C. Manolagas, J.M. Olefsky, eds.) pp. 1- 13. Churchhifl Livingstone, New York, NY, 1988.
13. Baron, R., Chakraborty, M., Chatlerjee, D., Horne, W., Lomri, A., Ravesloot, J.-H. Biology of the osteoclast. Iii "Physiology and Pharmacology of Bone" (G.R. Mundy T.J. Martin (eds.) 1993.
14. Rodan, G.A. Martin, T.J. Role of osteoblasts in hormonal control of bone resorption: a hypothesis., Calcif. Tissue Itit. 33:349-351, 1981.
15. Reeve, J., Meunier, P.J., Parsons, J.A., Bemat, M., Bijovet, O.L., Coupron, P., Edouard, C., Klenerman, L., Neer, R-M., Renier, J.C., Slovik, D., Vismqns, J. Potts, J.T. Anabofic effect of human parathyroid hormone fragment on trabecular bone in involutional osteoporosis: a multicentre trial. Br. Med J 280:1340-1344, 1980.
16. Liu, C.C., Kalu, D.N., Salerno, E., Echon, R-, Hollis, B.W., Ray, M. Pre-existing bone loss associated vath ovariectomy in rats is reversed by parathyroid hormone. J Bone Min. Res. 6:1071-1080, 199 1.
17. McCarthy, T.L., Centrella, M., Canalis, E. Parathyroid hormone enhances the transcript and polypeptide levels of insulin-like growth factor I in osteoblast enriched cultures from rat fetal bone. Endocrinology, 124:1247-1253, 1989.
18. Burger, H.C., van der Meer, J.W.M., Nijweide, P.J. Osteoclast formation from mononuclear phagocytes: Role of bone-forming cells. J Cell BioL 99:1901- 1906, 1984.
19. MacDonald, B.R.; Takahashi, N., McManus, L.M., Holahan, J., Mundy, G.R-, Roodman, G.D. Formation of multinucleated cells that respond to osteotropic hormones in long-term human bone marrow cultures. Endocrinology, 120:2326-2333, 1987.
20. Kalu, D.N. Ile ovariectomized rat model of postmenopausal bone loss. Bone MineraL 15:175-192, 1991.
21. Vanderschueren, D., Van Herck, E., Suiker, A.M.H., Visser, W.J., Schot, L.P.C., Chung, K, Lucas, R- S., Einhom, T.A. Bouillon, R. Bone and n3ineral metabolism in the androgen-resistant (testicular fe@ed) mate rat. J Bone Miner. Res., 8:801-809, 1993.
22. Bullock, L.P. Models of androgen insensitivity in the study of androgen action. Prog. Clin. Biol. Res. 94:365-379, 1982.
23. Zhang, R. Supowit, S.C., Klein, G.L., Lu, Z. Christensen, M.D., Lozano, R-, Simmons, D.J, Rat tall suspension reduces messenger-RNA level for growth factors and osteopontin and decreases the osteoblastic differentiation of bone marrow stromal cells. J. Bone Miner. Res. 10:415-423, 1995.
24. Machwate, M., Zerath, E., Holy, X., Hott, M., Modrowski, D., Malouvier, A., Marie, P.J. Skeletal unloading in rat decreases proliferation of rat bone and marrow-derived osteoblastic cells., Am. J Physiol. 264:E790-E799, 1993.
25. Jerome, C.P., Carlson, C.S., Register, T.C., Bain, F.T., Jayo, M.J., Weaver, D.S., Adams, M.R- Bone fimctional changes, in intact, ovariectomized, and ovariectomized, hormone-supplemented adult cynomolgus monkeys (macaca fascicularis) evaluated by senim markers and dynamic histomorphometry., J Bone Miner. Res. 9:527-540, 1994.
26. Jayo, M.J., Weaver, D.S., Adams, M.R., Rankin, S.E. Effects on bone of surgical monopause and estrogen therapy vhth or without progesterone replacement in Cynomolgus monkeys., Am. J Obstet. GynecoL 163:614-618, 1990.
27. Miller, L.C., Weaver, D.S., McAlister, J.A., Koritnik, D.K Effects of ovariectomy on vertebral trabecular bone in the cynomolgus monkey (macacafascicularis). Calcif. Tissue Int. 38:62-65, 1986.
28. Peng, Z., Tuukkanen, J., Zhang, H., Jamsa, T. Väänänen, H.K The mechanical strength of bone in different rat models of experimental osteoporosis. Bone, 15:523532, 1994.
29. Delmas, P.D. Biochemical markers of bone tumover in osteoporosis. Osteoporosis: Etiology, Diagnosis andmanagement, pp. 297-316, 1988.
30. Lazarus, H. M., Haynesworth, S.E., Gerson, S.L., Rosenthal, N.S., Caplan, A.I. Ex vivo expansion and subsequent infusion of human bone marrow-derived stromal progenitor ceils (mesenchymal progenitor cells): implications for therapeutic use. Bone Marrow Transplant. 16:557-564, 1995.
31. Thiede, M.A., Majumdar, M.P. NOer, S. Longin, K, Hardy, W., Gerson, S.L., Marshak, D.R. Storb, R., Sandmaier, B.M. Reinfiision of ex vivo-expanded, retrovirus-transduced autologous mesenchymal stem cells (MSCs) into irradiated dogs Blood, 86 (Suppl 1): II 2a, Abstract 43 3, 1995.

## Claims

1. Mesenchymal stem cells for use in treating osteoporosis in a mammal.

2. The mesenchymal stem cells according to claim 1 which are human mesenchymal stem cells.

3. The mesenchymal stem cells according to claim 2, wherein treatment of osteoporosis is by creating new mineralized bone tissue at critical sites in a regenerative tissue therapy.

4. The mesenchymal stem cells according to any one of claims 1 to 3, which are autologous cells.

5. An infusion preparation which comprises a suspension of purified culture-expanded autologous human mesenchymal stem cells in an infusion medium.

6. The infusion preparation according to claim 5 for use in treating osteoporosis.
